Europäisches Patentamt

European Patent Office

Office européen des brevets

(19)

(11) Veröffentlichungsnummer: **0 031 900**
**B1**

(12) **EUROPÄISCHE PATENTSCHRIFT**

(45) Veröffentlichungstag der Patentschrift:
31.07.85

(51) Int. Cl.⁴: **A 61 B 5/14**

(21) Anmeldenummer: **80107496.4**

(22) Anmeldetag: **01.12.80**

(54) Blutentnahmevorrichtung.

(30) Priorität: **04.12.79 DE 2948653**

(43) Veröffentlichungstag der Anmeldung:
**15.07.81 Patentblatt 81/28**

(45) Bekanntmachung des Hinweises auf die Patenterteilung:
**31.07.85 Patentblatt 85/31**

(84) Benannte Vertragsstaaten:
**AT BE CH FR GB IT LI LU NL SE**

(56) Entgegenhaltungen:
**EP - A - 0 017 728**
**DE - A - 1 812 742**
**DE - A - 2 903 167**
**FR - A - 2 187 361**
**FR - A - 2 292 456**
**FR - E - 90 721**
**US - A - 3 366 103**
**US - A - 4 166 450**

(73) Patentinhaber: **Sarstedt, Walter,
Kunststoff-Spritzgusswerk,
D-5223 Nümbrecht/Rommelsdorf (DE)**

(72) Erfinder: **Sarstedt, Walter, Kunststoff-Spritzgusswerk,
D-5223 Nümbrecht/Rommelsdorf (DE)**

(74) Vertreter: **Dipl.-Phys.Dr. Manitz Dipl.-Ing. Finsterwald
Dipl.-Ing. Grämkow Dipl.-Chem.Dr. Heyn
Dipl.-Phys.Rotermund, B.Sc. Morgan
Robert-Koch-Strasse 1, D-8000 München 22 (DE)**

## Beschreibung

Die Erfindung betrifft eine Blutentnahmevorrichtung nach dem Oberbegriff der Patentansprüche 1 oder 2.

Bei einer bekannten Blutentnahmevorrichtung dieser Art (DE-A-1 812 742) ist der elastische Verschlußstopfen unmittelbar am vorderen Ende des Entnahmeröhrchens eingesteckt. Die Führungshülse hat einen größeren Durchmesser als das Entnahmeröhrchen und wird entweder auf den Außenmantel des Entnahmeröhrchens aufgeschoben oder auf einen Gewindevorsprung des Verschlußstopfens aufgeschraubt. Bei der bekannten Blutentnahmevorrichtung muß also der in das vordere Ende des Entnahmeröhrchens eingedrückte Verschlußstopfen herausgenommen werden, wenn der Inhalt des Entnahmeröhrchens zur weiteren Verarbeitung zugänglich sein soll. Da der Verschlußstopfen nun aufgrund der für das Selbstschließen nach dem Durchstechen erforderlichen elastischen Eigenschaften stark klemmend in dem Entnahmeröhrchen sitzt, kann er nur unter elastischer Verformung ruckartig von der Vorderseite des Entnahmeröhrchens abgenommen werden, wobei es in unerwünschter Weise zum Verspritzen des in dem Entnahmeröhrchen befindlichen Blutes kommen kann. Außerdem ist es bei der bekannten Vorrichtung nachteilig, daß die die Kanüle enthaltende Führungshülse mit ihrem Innendurchmesser an den Außendurchmesser des Entnahmeröhrchens angepaßt sein muß und so die Führungshülse wesentlich voluminöser und platzaufwendiger gebaut sein muß, als dies für ihre Funktion zur Aufnahme und Halterung der Kanüle eigentlich nötig wäre.

Weiter ist bereits ein Entnahmeröhrchen mit einer Schraubkappe bekannt (FR-A-2 292 456), von der axial ein mit einer zerreißbaren Folie verschlossener konischer Anschlußstutzen vorsteht, der zum flüssigkeitsdichten Aufsetzen einer Kanüle oder eines Deckels dient, welcher aber zur axialen Führung einer Führungshülse weder geeignet noch bestimmt ist.

Bei einem weiteren bekannten Entnahmeröhrchen mit aufschraubbarer Verschlußkappe (US-PS 4 166 450) wird die Führungshülse am Außenumfang der Verschlußkappe geführt, was nicht nur deswegen nachteilig ist, weil die Führungshülse den gleichen Durchmesser wie die Verschlußkappe aufweisen muß und deswegen sehr voluminös ist, sondern weil die Länge der Führungsfläche durch die Länge der Verschlußkappe begrenzt ist. Außerdem muß für die Führung der Führungshülse gerade diejenige Fläche der Verschlußkappe verwendet werden, die zum Erfassen mit den Fingern aufgerauht oder mit einer reibungserhöhenden Oberfläche versehen sein soll.

Die Aufgabe der vorliegenden Erfindung besteht nun darin, eine Blutentnahmevorrichtung der eingangs genannten Gattung zu schaffen, die unter einwandfreier Führung und geringem Platzbedarf der Führungshülse ein bequemes und sauberes Arbeiten während der Blutentnahme und bei der anschließenden Weiterverarbeitung der entnommenen Blutprobe ermöglicht.

Zur Lösung dieser Aufgabe sind die Merkmale der Patentansprüche 1 oder 2 vorgesehen.

Aufgrund dieser Ausbildung sind die Funktionen des Öffnens des Entnahmeröhrchens nach einer Blutentnahme und des Durchstechens des Verschlußstopfens zu Beginn der Blutentnahme mittels der Kanüle so in einem Bauteil vereinigt, daß die Verschlußkappe nach dem Gesichtspunkt eines dichten Verschlusses und einer leichten Abnehmbarkeit vom Entnahmeröhrchen und der Verschlußstopfen speziell im Hinblick auf seine Funktion als von der Kanüle durchstechbares, jedoch nach dem Herausziehen der Kanüle selbstschließendes Bauglied ausgebildet werden kann. Daraus resultiert die Möglichkeit, daß sowohl der Ansatz mit dem darin angeordneten Verschlußstopfen als auch die Führungshülse ohne Rücksicht auf den Durchmesser des Entnahmeröhrchens dimensioniert werden können. Der Ansatz, der Verschlußstopfen und die Führungshülse können also einen erheblich geringeren Durchmesser als das Entnahmeröhrchen aufweisen.

Umgekehrt kann die Verschlußkappe ohne Beachtung der Abmessungen der Führungshülse und des Ansatzes dem Durchmesser jedes beliebigen Entnahmeröhrchens angepaßt werden. Die Verschlußkappe und der Ansatz stellen also praktisch einen Adapter dar, mit dem eine einen einheitlichen Durchmesser aufweisende Führungshülse verwendet werden kann, wobei jeweils nur eine dem Durchmesser des Entnahmeröhrchens angepaßte Verschlußkappe verwendet werden muß, während der Ansatz und die Führungshülse ihre Standardabmessungen behalten.

Aufgrund der zylindrischen Form des Ansatzes wird während des Aufschiebens auf den bzw. Abziehens von dem Ansatz eine einwandfreie Führung der Führungshülse insbesondere während des Durchstechens des Verschlußstopfens erzielt, was wichtig ist, um eine sich beim Herausziehen der Kanüle wieder von selbst schließende saubere Durchstechöffnung zu erzielen. Aufgrund der Herstellung der Einzelteile der erfindungsgemäßen Blutentnahmevorrichtung nach dem Spritzgußverfahren sind durch das Erfordernis des Ausformens bedingte, geringfügige Abweichungen von der exakten Zylinderform möglich, welche jedoch die sichere Führung der Führungshülse am Ansatz nicht beeinträchtigen.

Besonders vorteilhaft ist es, wenn der Ansatz exzentrisch zur Verschlußkappe angeordnet ist, wobei der Außendurchmesser der Führungshülse und die Exzentrizität so abgestimmt sein sollen, daß die Führungshülse radial nicht über den Umfang der Verschlußkappe vorsteht.

Aufgrund dieser Ausbildung kann die Blutentnahmevorrichtung glatt auf den Unterarm des Patienten oder eine andere Stelle, an der Blut entnommen werden soll, aufgelegt werden.

Um sich möglichst nahe dem hinteren Ende der Kanüle zu befinden, soll der Verschlußstopfen zweckmäßig in das vordere Ende des Ansatzes eingesetzt sein.

Eine weitere vorteilhafte Ausführungsform ist so ausgebildet, daß der Verschlußstopfen eine dünne Scheibe ist, die gegen Durchbiegung an ihrer Hinterseite von einem oder mehreren einwärts vorstehenden Vorsprüngen abgestützt ist. Diese Ausbildung ist möglich, da aufgrund der erfindungsgemäßen Ausbildung der Verschlußstopfen einen festen Bestandteil des Ansatzes und damit der Verschlußkappe bildet. Die Ausbildung des Verschlußstopfens in Form einer dünnen Scheibe ist auch deswegen ohne weiteres möglich, weil sowohl der Ansatz als auch die Scheibe mit einem so geringen Durchmesser ausgebildet sein können, wie das für eine einwandfreie Führung der Führungshülse gerade noch erfinderisch ist.

Eine einwandfreie Halterung der dünnen Scheibe kann nach einer ersten baulichen Ausführungsform dadurch erzielt werden, daß eine starre, mit einer Mittelbohrung versehene, fest mit dem oberen Rand des Ansatzes verbundene Haltescheibe auf den scheibenförmigen Verschlußstopfen aufgesetzt ist. Die Halterung der dünnen Scheibe kann aber auch dadurch verwirklicht werden, daß der obere Rand eines am vorderen Ende des Ansatzes vorgesehenen Kragens über die Scheibe einwärts umgebördelt ist.

Sofern in dem Entnahmeröhrchen ein chemisches Reagens enthalten ist, das bei längerem Lagern den aus einem weichelastischen Werkstoff bestehenden Verschlußstopfen angreifen, beispielsweise anlösen oder aufquellen könnte, ist hinter dem Verschlußstopfen eine den Durchlaß verschließende Folie angeordnet. Die Folie schützt somit die Hinterseite des Verschlußstopfens gegen Beschädigung. Die Folie wird ebenso wie der Verschlußstopfen selbst beim Gebrauch durch das hintere Kanülenende durchstoßen.

Eine besonders gute Dichtheit wird erzielt, wenn die Folie einstückig mit dem Ansatz ausgebildet ist.

Im übrigen ist es zweckmäßig, wenn der Verschlußstopfen unter radialer Vorspannung in den Ansatz eingelegt ist, damit er sich nach dem Herausziehen des Kanülenendes wieder von selbst schließt.

Im allgemeinen ist die Kanüle unlösbar mit der Führungshülse verbunden, so daß sie nach Gebrauch mit dieser fortgeworfen wird. Die Herstellung der Führungshülse als Wegwerfteil ist wegen der erfindungsgemäß ermöglichenden geringen Größe und der damit verbundenen wirtschaftlichen Herstellung ohne weiteres möglich.

Gegebenenfalls kann aber die Kanüle auch herausnehmbar in der Führungshülse angeordnet sein, was beispielsweise mittels eines Gewindes erfolgen kann. In einem derartigen Fall soll nur die Führungshülse nach Gebrauch weggeworfen werden, während die kostspieligere Kanüle wieder verwendet werden könnte.

Die erfindungsgemäße Vorrichtung gestattet auch in einfacher Weise die Anordnung eines an sich bekannten Verschlusses für das hintere Ende der doppelendigen Kanüle und dadurch einen Verschluß der Kanüle, deren vorderes Ende in der Vene verbleibt, während eines Wechsels des Entnahmeröhrchens. Zu diesem Zweck kann auf das hintere Kanülenende ein an seinem Ende verschlossener dünner Schlauch aus einem weichelastischen Werkstoff aufgeschoben sein. Beim Zurückschieben der Führungshülse auf den Ansatz der Verschlußkappe durchsticht das hintere, angeschärfte Kanülenende zunächst das hintere Ende dieses dünnen Schlauches und erst anschließend den Verschlußstopfen im Ansatz. Der Schlauch selbst schiebt sich dabei harmonikaartig zusammen. Wenn dann nach Füllung des angeschlossenen Entnahmeröhrchens der Ansatz wieder aus der Führungshülse herausgezogen wird, schiebt sich der Schlauch wieder auseinander und das durchstochene untere Ende des Schlauches schließt sich wieder und verschließt damit das hintere Kanülenende.

Vorzugsweise ist dabei vorgesehen, daß die Haftung zwischen Führungshülse und Ansatz nur so groß ist, daß ein Abschieben der Führungshülse vom Ansatz durch den zusammengedrückten Schlauch gerade verhindert wird. Damit ist sichergestellt, daß die zurückgeschobene Führungshülse so lange in ihrer Lage verbleibt, bis sie wieder von Hand abgeschoben wird.

Die Arbeitsweise der erfindungsgemäßen Blutentnahmevorrichtung ist wie folgt:

Das verschlossene Entnahmeröhrchen, das unter Umständen eine kleine Menge eines besonderen Reagenz in fester oder flüssiger Form enthält, wird der Packung entnommen und eine ebenfalls entnommene sterile Führungshülse mit Kanüle auf den Ansatz der Verschlußkappe des Entnahmeröhrchens aufgeschoben. Nachdem die Führungshülse zunächst nur so weit auf den Ansatz aufgeschoben wurde, daß sie von diesem fest und sicher geführt wird, wird dann, unter Festhalten der Führungshülse mit Daumen und Zeigefinger, das vordere, freistehende Ende der Kanüle in die Vene eingeführt. Sodann wird unter Beibehaltung der Lage der Führungshülse und der Kanüle des Entnahmeröhrchens mit seinem Ansatz vorwärtsbewegt. Dabei durchsticht das hintere, in der Führungshülse befindliche Ende der Kanüle den Verschlußstopfen und stellt nunmehr eine Verbindung zwischen der Vene über die Kanüle mit dem Innenraum des Entnahmeröhrchens her. Erst jetzt, nachdem sich alle Teile in ihrer richtigen Lage befinden, wird der Kolben, je nach Konstruktion, mechanisch oder pneumatisch, langsam zurückgezogen, und damit die Blutprobe in schonender Weise entnommen.

Die Erfindung wird im folgenden beispielsweise anhand der Zeichnung beschrieben; in dieser zeigt

Fig. 1 eine Seitenansicht einer erfindungsgemäßen Blutentnahmevorrichtung,

Fig. 2 einen vergrößerten Axialschnitt der Verschlußkappe, des Ansatzes, der Führungshülse und der Kanüle der in Fig. 1 gezeigten Blutentnahmevorrichtung,

Fig. 3 einen Schnitt der Verschlußkappe und des Ansatzes einer weiteren Ausführungsform einer erfindungsgemäßen Blutentnahmevorrichtung,

Fig. 4 einen Schnitt nach Linie IV-IV in Fig. 3,

Fig. 5 eine teilweise axial geschnittene vergrößerte Seitenansicht der Verschlußkappe und der daran angeordneten Bauteile einer weiteren Ausführungsform der erfindungsgemäßen Blutentnahmevorrichtung,

Fig. 6 den gleichen Schnitt wie Fig. 5, jedoch unter Weglassung der Verschlußkappe und Darstellung der Führungshülse während des Durchstechens des Verschlußstopfens,

Fig. 7 eine vergrößerte Teilschnittansicht des vorderen Endes des Ansatzes einer weiteren Ausführungsform der erfindungsgemäßen Blutentnahmevorrichtung, und

Fig. 8 einen Teilschnitt einer weiteren Ausführungsform des Ansatzes, welcher in das hintere Ende der Führungshülse eingesetzt ist.

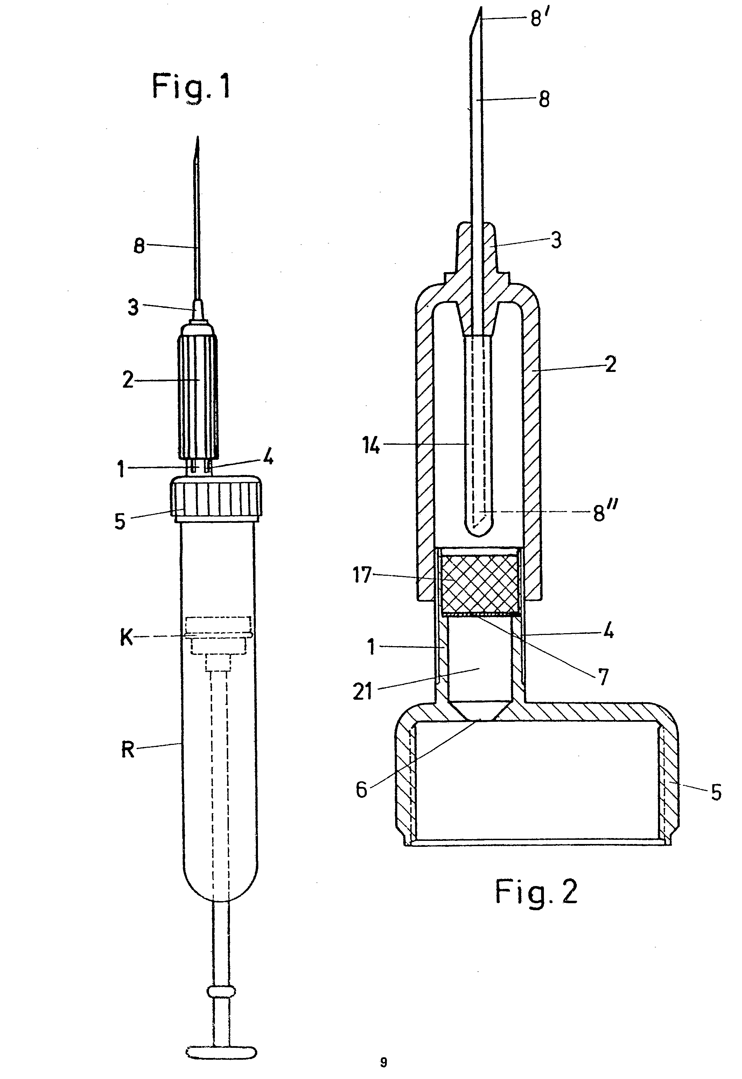

Die in den Fig. 1 und 2 dargestellte Blutentnahmevorrichtung weist ein zylindrisches Entnahmeröhrchen R mit einem darin luftdicht verschiebbaren, von einer Kolbenstange betätigbaren Kolben K, eine Verschlußkappe 5, die auf ein Außengewinde des Entnahmeröhrchens R aufgeschraubt ist, und eine auf einen Ansatz 1 der Verschlußkappe 5 aufgeschobene Führungshülse 2 auf, die in einem Stutzen 3 eine doppelendige Kanüle 8 trägt. Sowohl die Verschlußkappe 5 mit dem Ansatz 1 als auch die Führungshülse 2 mit dem Stutzen 3 bestehen aus thermoplastischem Kunststoff. Das vordere Ende 8' und das hintere Ende 8'' der Kanüle 8 sind angeschärft, und der in die Führungshülse 2 hineinragende hintere Teil der Kanüle 8 ist von einem unten geschlossenen Schlauch 14 aus Weichgummi überzogen und dadurch allseits dicht umhüllt.

Die Verschlußkappe 5 besitzt exzentrisch zu ihrer Mittelachse einen engen Durchlaß 6, der sich nach oben trichterförmig erweitert und in den zylindrischen Innenraum 21 eines an die Vorderseite der Verschlußkappe 5 anschließenden Ansatzes 1 übergeht. In seinem oberen Teil erweitert sich der Innenraum 21 und nimmt dort einen Verschlußstopfen 17 aus Weichgummi auf, der unter radialer Vorspannung in den Ansatz 1 eingesetzt ist. Die äußere Mantelfläche des Ansatzes 1 ist von einer oder mehreren, achsparallel verlaufenden Nuten 4 durchzogen, die der Entlüftung dienen. Wahlweise können statt der Nuten auch Führungsrippen vorgesehen sein.

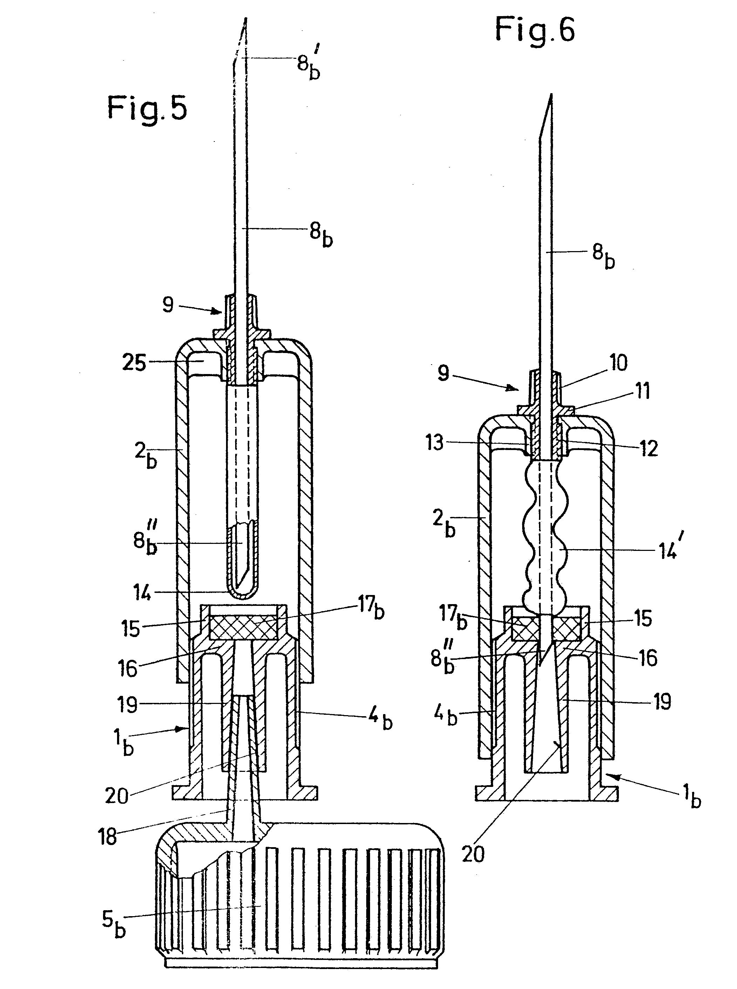

Nach dem Einführen des vorderen Endes 8' der Kanüle in die Vene wird die Führungshülse 2 relativ zum Körper des Patienten festgehalten, während das Entnahmeröhrchen R mit seiner Verschlußkappe 5 und dem davon vorstehenden Ansatz 1 in Richtung auf die Führungshülse 2 vorgeschoben wird. Dabei durchbohrt das hintere Ende 8'' der Kanüle zunächst das untere Ende des Gummischlauches 14 und dann den Verschlußstopfen 17. Dabei schiebt sich, wie man es in Fig. 6 für eine andere Ausführungsform erkennt, dieser Schlauch harmonikaartig zusammen.

Unter dem Verschlußstopfen 17 liegt eine Folie 7 aus Kunststoff, die ebenfalls von dem hinteren Ende 8'' der Kanüle beim Zusammenschieben der beiden Teile durchstochen wird.

In dieser Position wird der Kolben K zurückgezogen, wodurch im Innenraum 21 ein Vakuum entsteht und aus der Vene Blut durch die Kanüle 8 in den Innenraum 21 des Ansatzes 1 und von dort in das Entnahmeröhrchen R einfließt.

Nach erfolgter Blutentnahme wird dann die gesamte Blutentnahmevorrichtung abgenommen, indem das vordere Ende 8' der Kanüle aus der Vene entfernt wird. Sodann wird die Führungshülse 2 mit der Kanüle 8 vom Ansatz 1 abgezogen und fortgeworfen. Der Verschlußstopfen 17 schließt sich infolge seiner radialen Vorspannung wieder und die Blutprobe ist sicher im Entnahmeröhrchen R aufbewahrt.

Falls man jedoch ein zweites oder drittes Entnahmeröhrchen mit Blut desselben Patienten füllen möchte, beläßt man das vordere Ende 8' der Kanüle in der Vene, hälte also die Führungshülse 2 am Körper des Patenten fest, und entfernt lediglich das Entnahmeröhrchen zusammen mit der Verschlußkappe 5 und dem Ansatz 1. Dabei schiebt sich der Schlauch 14 wieder über das Ende 8'' der Kanüle 8 und schließt sich elastisch ebenso wie der Verschlußstopfen 17. Dadurch wird das hintere Ende 8'' der Kanüle verschlossen, und es kann kein Blut aus der Kanüle 8 austreten, bis ein zweites, leeres Entnahmeröhrchen R mit aufgeschraubter Verschlußkappe 5 angesetzt und mit seinem Ansatz 1 in die Führungshülse 2 eingeschoben wird.

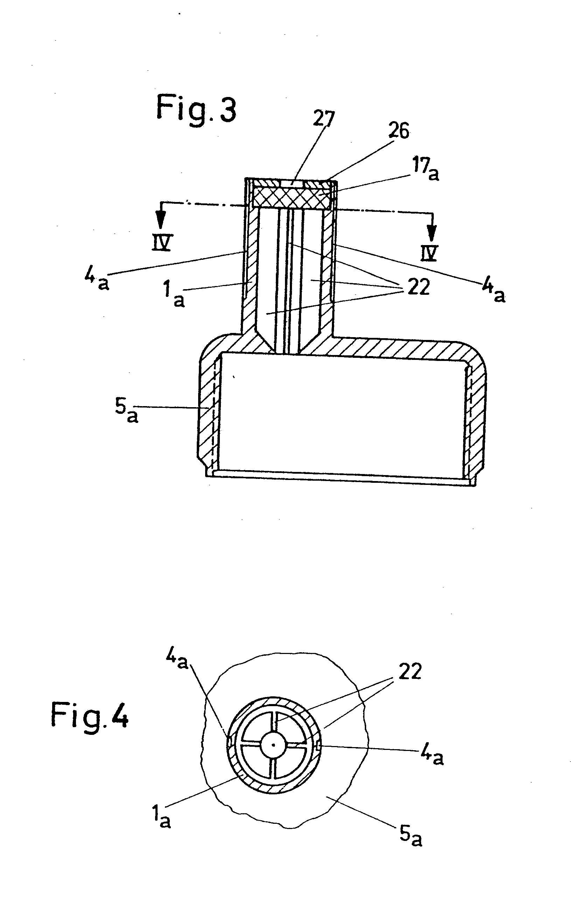

Bei der in Fig. 3 und 4 dargestellten abgewandelten Ausführungsform der Verschlußkappe 5a und des Ansatzes 1a mit den Nuten 4a wird ein Verschlußstopfen 17a in Form einer dünnen Scheibe aus Weichgummi verwendet. Zur Unterstützung dieser Scheibe aus Weichgummi gegen unzulässige Durchbiegung sind vier flügelartige Vorsprünge 22 vorgesehen, die vom Ansatz 1a radial einwärts vorstehen. Die Vorsprünge 22 lassen einen engen zylindrischen Raum in der Mitte des Ansatzes 1a frei, in den das hintere Ende der Kanüle 8 eintreten kann.

Auf den Verschlußstopfen 17a ist eine starre Scheibe 26 mit einer Mittelbohrung 27 aufgelegt, die ebenso, wie die Verschlußkappe 5a und der Ansatz 1a aus Kunststoff besteht und nach dem Einlegen fest mit dem oberen Rand des Ansatzes 1a verbunden wird, so daß beim Einstechen und anschließenden Herausziehen einer Kanüle 8 nicht die Gefahr besteht, daß der scheibenförmige Verschlußstopfen 17a aus dem Ansatz 1a herausgezogen wird.

Die Blutentnahmevorrichtung nach den Fig. 5 und 6 ist gegenüber den vorstehend beschriebenen Ausführungen so abgewandelt, daß man sie in Verbindung mit bekannten Blutentnahmevorrichtungen, beispielsweise Injektionsspritzen mit konischem Anschlußstutzen 18 verwenden kann. Wie in Fig. 5 angedeutet ist, befindet sich auf der Verschlußkappe 5b einer solchen bekannten Blutentnahmevorrichtung ein konischer Anschlußstutzen 18, der üblicherweise zum Aufstecken des konischen Anschlußtrichters einer Kanüle vorgesehen ist. Anstelle eines solchen Anschlußtrichters ist jedoch bei dem Ausführungsbeispiel nach den Fig. 5 und 6 ein besonders ausgebildeter Ansatz 1b aufgesteckt, und zwar mittels eines von seiner vorderen Querwand 16 nach hinten vorstehenden und sich in seinen Innenraum erstreckenden Rohrstutzens 19 mit konischer Bohrung 20, die zu dem konischen Anschlußstutzen 18 paßt. Von der Querwand 16 des Ansatzes 1b steht ein verengter Kragen 15 nach vorn vor, in den eine dünne Scheibe 17b aus Weichgummi als Verschlußstopfen eingesetzt ist. Der äußere Mantel des Ansatzes 1b ist mit Längsnuten 4b versehen, die beim Einschieben des Ansatzes in die zugehörige Führungshülse 2b eine Entlüftung bewirken.

Die Führungshülse 2b ist an ihrem vorderen Ende verschlossen und trägt dort einen einspringenden Hals 13 (Fig. 6) mit Innengewinde, in den ein besonderer Kanülenhalter 9 mit einem Außengewinde 12 eingeschraubt ist. Das Einschrauben wird durch Längsrippen 10 erleichtert und durch einen Flansch 11 begrenzt, der sich auf die vordere Stirnwand der Führungshülse 2b legt. Zur Verstärkung befinden sich zwischen dem Hals 13 und dem Mantel der Führungshülse 2b radiale Rippen 25.

Die in den Halter 9 eingesetzte Kanüle 8b wird mit ihrem vorderen Ende 8b' in die Vene eingeführt. Das hintere Ende 8b'' ist wieder von dem hinten geschlossenen Schlauch 14 aus Weichgummi überzogen und allseits eingehüllt. Der Schlauch 14 wird, wenn die Führungshülse 2b gegenüber dem Kragen 1b in die in Fig. 6 dargestellte Position verschoben wird, vom hinteren Ende 8b'' der Kanüle durchstochen und verformt sich harmonikaartig, wie das bei 14' in Fig. 6 gezeigt ist.

Wenn man dann die Führungshülse 2b und den Ansatz 1b wieder auseinanderzieht, streckt sich der Schlauch 14 wieder in seine in Fig. 5 dargestellte Lage und verschließt das hintere Ende 8b'' der Kanüle.

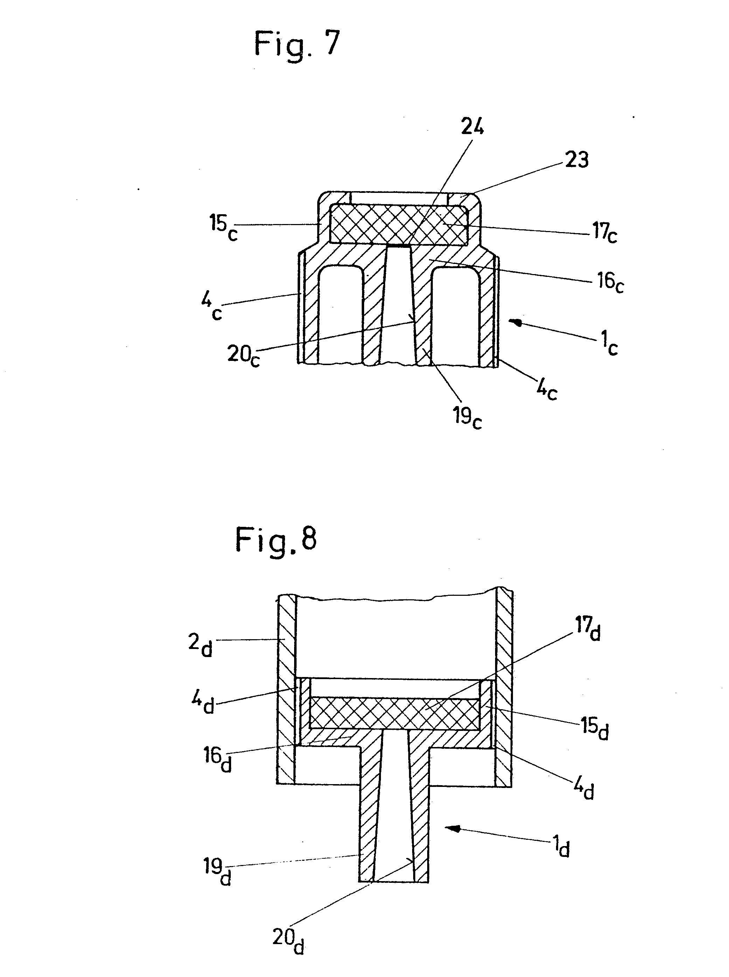

Bei der abgewandelten Ausführungsform nach Fig. 7 ist der vordere Rand 15c des Ansatzes 1c bei 23 radial einwärts umgebogen und hält damit den Verschlußstopfen 17c fest in seiner Lage auf einem am Ansatz 1c vorgesehenen radial nach innen gerichteten Vorsprung 16c.

Das vordere Ende der konischen Bohrung 20c des Rohrstutzens 19c ist durch eine Folie 24 verschlossen, die bei der Herstellung des Ansatzes 1c aus thermoplastischem Kunststoff mit angespritzt wird. Am äußeren Mantel des Ansatzes 1c sind zur Entlüftung Längsnuten 4c vorgesehen.

Fig. 8 zeigte eine weitere Ausführungsform des Ansatzes 1d, welcher in das hintere Ende der Führungshülse 2d eingeschoben ist. Zur Aufnahme des Verschlußstopfens 17d ist ein kurzer Zylinder 15d mit äußeren Längsnuten 4d vorgesehen, der an seiner hinteren Seite durch eine Stirnwand 16d mit einer Mittelbohrung abgeschlossen ist, an die sich nach hinten ein Rohrstutzen 19d mit konischer Bohrung 20d anschließt.

Anstelle der Nuten können zur Entlüftung der Führungshülse achsparallele Führungsrippen vorgesehen werden.

## Patentansprüche

1. Blutentnahmevorrichtung, die ein zylindrisches Entnahmeröhrchen und einen mit dem vorderen Ende des Entnahmeröhrchens in Verbindung stehenden durchstechbaren, nach dem Durchstechen selbstschließenden Verschlußstopfen sowie eine im Bereich des Verschlußstopfens aufschiebbare rohrförmige Führungshülse aufweist, die an ihrem vorderen Ende eine doppelendige, beiderseits angeschärfte Kanüle trägt, deren aus der Führungshülse vorstehendes Ende zur Einführung in ein Blutgefäß vorgesehen ist, während ihr hinteres Ende soweit in die Führungshülse hineinragt, daß es beim Aufschieben der Führungshülse den Verschlußstopfen durchsticht, wobei mittels eines durch einen in dem Entnahmeröhrchen angeordneten Kolben erzeugten Vakuums Blut durch die Kanüle in das Entnahmeröhrchen gesaugt wird, dadurch gekennzeichnet, daß die Führungshülse (2) auf einen von einer auf das Entnahmeröhrchen (R) aufsteckbaren oder aufschraubbaren Verschlußkappe (5, 5a) axial vorstehenden, den Verschlußstopfen (17, 17a) enthaltenden Ansatz (1, 1a) aufgeschoben ist, welcher zylindrisch ausgebildet ist und zur Entlüftung der Führungshülse (2) achsparallele Führungsrippen oder Nuten (4, 4a) aufweist.

2. Blutentnahmevorrichtung, die ein zylindrisches Entnahmeröhrchen und einen mit dem vorderen Ende des Entnahmeröhrchens in Verbindung stehenden durchstechbaren, nach dem Durchstechen selbstschließenden Verschlußstopfen sowie eine im Bereich des Verschlußstopfens aufschiebbare rohrförmige Führungshülse aufweist, die an ihrem vorderen Ende eine doppelendige, beiderseits angeschärfte Kanüle trägt, deren aus der Führungshülse vorstehendes Ende zur Einführung in ein Blutgefäß vorgesehen ist, während ihr hinteres Ende soweit in die Führungshülse hineinragt, daß es beim Aufschieben der Führungshülse den Verschlußstopfen durchsticht, wobei mittels eines durch einen in dem Entnahmeröhrchen angeordneten Kolben erzeugten Vakuums Blut durch die Kanüle in das Entnahmeröhrchen gesaugt wird, dadurch gekennzeichnet, daß die Führungshülse (2b) auf einen den Verschlußstopfen (17b, 17c, 17d) enthaltenden Ansatz (1b, 1c, 1d) aufgeschoben ist, welcher als Zwischenstück auf einen von einer auf das Entnahmeröhrchen (R) aufsteckbaren oder aufschraubbaren Verschlußkappe (5b) axial vorstehenden konischen Anschlußstutzen (18) mittels einer entsprechend konischen Bohrung (20, 20c, 20d) aufgesetzt ist, zylindrisch ausgebildet ist und zur Entlüftung der Führungshülse (2b) achsparallele Führungsrippen oder Nuten (4b, 4c, 4d) aufweist.

3. Vorrichtung nach Anspruch 1 oder 2, dadurch gekennzeichnet, daß der Ansatz (1, 1b, 1c, 1d) exzentrisch zur Verschlußkappe (5) angeordnet ist.

4. Vorrichtung nach Anspruch 3, dadurch gekennzeichnet, daß der Außendurchmesser der Führungshülse (2) und die Exzentrizität so abgestimmt sind, daß die Führungshülse (2) radial nicht über den Umfang der Verschlußkappe vorsteht.

5. Vorrichtung nach einem der vorhergehenden Ansprüche, dadurch gekennzeichnet, daß der Verschlußstopfen (17, 17a, 17b, 17c, 17d) in das vordere Ende des Ansatzes (1, 1a, 1b, 1c, 1d) eingesetzt ist.

6. Vorrichtung nach einem der vorhergehenden Ansprüche, dadurch gekennzeichnet, daß der Verschlußstopfen eine dünne Scheibe (17a, 17b, 17c, 17d) ist, die gegen Durchbiegung an ihrer Hinterseite von einem oder mehreren einwärts vorstehenden Vorsprüngen (16, 16c, 16d, 22) abgestützt ist und daß eine starre, mit einer Mittelbohrung (27) versehene, fest mit dem oberen Rand des Ansatzes (1a) verbundene Haltescheibe (26) auf den scheibenförmigen Verschlußstopfen (17a) aufgesetzt ist.

7. Vorrichtung nach Anspruch 6, dadurch gekennzeichnet, daß der obere Rand (23) eines am vorderen Ende des Ansatzes (1c) vorgesehenen Kragens (15c) über die Scheibe (17c) einwärts umgebördelt ist.

8. Vorrichtung nach einem der vorhergehenden Ansprüche, dadurch gekennzeichnet, daß hinter dem Verschlußstopfen (17, 17c) eine den Durchlaß verschließende Folie (7, 24) angeordnet ist.

9. Vorrichtung nach Anspruch 8, dadurch gekennzeichnet, daß die Folie (24) einstückig mit dem Ansatz (1c) ausgebildet ist.

**Claims**

1. A blood extraction device having a cylindrical extraction tube and a puncturable closure plug which stands in connection with the front end of the extraction tube and is self closing after being punctured, and also a tubular guide sleeve which can be pushed into place in the vicinity of the closure plug, wherein the tubular guide sleeve carries at its front ent a double ended cannula which is sharpened at both ends, with the end of the cannula which projects out of the guide sleeve being provided for insertion into a blood vessel, while its rear end extends sufficiently far into the guide sleeve that it penetrates the closure plug on pushing the guide sleeve into place, with blood being sucked through the cannula into the extraction tube by means of a vacuum generated by a piston arranged in the extraction tube, characterized in that the guide sleeve (2) is pushed into place on a projection (1, 1a) which projects axially forwardly from a closure cap (5, 5a) capable of being plugged or screwed onto the extraction tube (R), with the projection containing the closure plug (17, 17a), being of cylindrical construction and having axially parallel guide ribs or grooves (4, 4a) for venting the guide sleeve (2).

2. A blood extraction device having a cylindrical extraction tube and a puncturable closure plug which stands in connection with the front end of the extraction tube and is self closing after being punctured, and also a tubular guide sleeve which can be pushed into place in the vicinity of the closure plug, wherein the tubular guide sleeve carries at its front end a double ended cannula which is sharpened at both ends, with the end of the cannula which projects out of the guide sleeve being provided for insertion into a blood vessel, while its rear end extends sufficiently far into the guide sleeve that it penetrates the closure plug on pushing the guide sleeve into place, with blood being sucked through the cannula into the extraction tube by means of a vacuum generated by a piston arranged in the extraction tube, characterized in that the guide sleeve (2b) is slid into place on a projection (1b, 1c, 1d) which contains the closure plug (17b, 17c, 17d) and which is mounted as an intermediate member on a conical connection spigot (18), by means of a corresponding conical bore (20, 20c, 20d), with the conical connection spigot projecting axially forwardly from a closure cap (5b) capable of being plugged or screwed onto the extraction tube (R), and with the projection being of cylindrical construction and having axially parallel guide ribs or grooves (4b, 4c, 4d) to vent the guide sleeve (2b).

3. Apparatus in accordance with claim 1 or claim 2, characterized in that the projection (1, 1b, 1c, 1d) is arranged eccentrically relative to the closure cap (5).

4. Apparatus in accordance with claim 3, characterized in that the external diameter of the guide sleeve (2) and the eccentricity are so matched that the guide sleeve (2) does not project radially beyond the periphery of the closure cap.

5. Apparatus in accordance with one of the preceeding claims, characterized in that the closure plug (17, 17a, 17b, 17c, 17d) is inserted into the front end of the projection (1, 1a, 1b, 1c, 1d).

6. Apparatus in accordance with one of the preceeding claims, characterized in that the closure plug is a thin disc (17a, 17b, 17c, 17d) which is braced against bending at its rear side by one or more inwardly projecting projections (16, 16c, 16d, 22); and in that a rigid retaining disc (26) provided with a central bore (27) and fixedly connected with the upper edge of the projection (1a) is mounted on the disc-like closure plug (17a).

7. Apparatus in accordance with claim 6, characterized in that the upper edge (23) of a collar (15c) provided at the front end of the projection (1c) is turned inwardly over the disc (17c).

8. Apparatus in accordance with one of the preceeding claims, characterized in that a foil (7, 24) which closes the passage is arranged behind the closure plug (17, 17c).

9. Apparatus in accordance with claim 8, characterized in that the foil (24) is formed in one piece with the projection (1c).

## Revendications

1. Dispositif de prélèvement sanguin, qui présente un petit tube de prélèvement cylindrique et un bouchon d'obturation perçable s'auto-obturant après la percée, se trouvant en liaison avec l'extrémité avant du tube de prélèvement, ainsi qu'une douille de guidage cylindrique pouvant être enfilée au voisinage du bouchon d'obturation, douille qui porte à son extrémité avant une canule à double extrémité, affûtée de part et d'autre, dont l'extrémité faisant saillie à partir de la douille de guidage est prévue pour l'introduction dans un vaisseau sanguin, tandis que son extrémité arrière pénètre tellement loin dans la douille de guidage qu'elle perce le bouchon d'obturation lors de l'enfilage de la douille de guidage, du sang étant aspiré dans le petit tube de prélèvement, à travers la canule, grâce à un vide engendré par un piston disposé dans le petit tube de prélèvement, caractérisé en ce que la douille de guidage (2) est glissée sur un appendice (1, 1a) faisant saillie axialement d'un capuchon de fermeture (5 , 5a) pouvant être enfoncé ou vissé sur le petit tube de prélèvement (R), et contenant le bouchon d'obturation (17, 17a), appendice qui est de réalisation cylindrique et présente des nervures de guidage ou gorges (4, 4a) parallèles à l'axe pour l'aération de la douille de guidage (2).

2. Dispositif de prélèvement sanguin, qui présente un petit tube de prélèvement cylindrique et un bouchon d'obturation perçable s'auto-obturant après la percée, se trouvant en liaison avec l'extrémité avant du tube de prélèvement, ainsi qu'une douille de guidage cylindrique pouvant être enfilée au voisinage du bouchon d'obturation, douille qui porte à son extrémité avant une canule à double extrémité, affûtée de part et d'autre, dont l'extrémité faisant saillie à partir de la douille de guidage est prévue pour l'introduction dans un vaisseau sanguin, tandis que son extrémité arrière pénètre tellement loin dans la douille de guidage qu'elle perce le bouchon d'obturation lors de l'enfilage de la douille de guidage, du sang étant aspiré dans le petit tube de prélèvement, à travers la canule, grâce à un vide engendré par un piston disposé dans le petit tube de prélèvement, caractérisé en ce que la douille de guidage (2b) est glissée sur un appendice (1b, 1c, 1d) contenant le bouchon d'aspiration (17b, 17c, 17d), qui est placé en tant que pièce intermédiaire, au moyen d'un alésage conique approprié (20, 20c, 20d), sur un ajutage conique (18) faisant saillie axialement sur un capuchon d'obturation (5b) pouvant être enfoncé ou vissé sur le petit tube de prélèvement (R), est de réalisation cylindrique, et présente des nervures de guidage ou gorges (4b, 4c, 4d) parallèles à l'axe pour l'aération de la douille de guidage (2b).

3. Dispositif suivant la revendication 1 ou 2, caractérisé en ce que l'appendice (1, 1b, 1c, 1d) est disposé excentriquement par rapport au capuchon d'obturation (5).

4. Dispositif suivant la revendication 3, caractérisé en ce que le diamètre externe de la douille de guidage (2) et l'excentricité sont accordés de telle sorte que la douille de guidage (2) ne fait pas saillie radialement au-delà de la périphérie du capuchon d'obturation.

5. Dispositif suivant l'une des revendications précédentes, caractérisé en ce que le bouchon d'obturation (17, 17a, 17b, 17c, 17d) est placé dans l'extrémité avant de l'appendice (1, 1a, 1b, 1c, 1d).

6. Dispositif suivant l'une des revendications précédentes, caractérisé en ce que le bouchon d'obturation est un mince disque (17a, 17b, 17c, 17d), qui est soutenu contre une flexion sur sa face arrière, par une ou plusieurs saillies s'étendant vers l'intérieur (16, 16c, 16d, 22), et en ce qu'un disque de retenue rigide (26), doté d'un alésage central (27), relié rigidement au bord supérieur de l'appendice (1a), est placé sur le bouchon d'obturation en forme de disque (17a).

7. Dispositif suivant la revendication 6, caractérisé en ce que le bord supérieur (23) d'un collet (15c) prévu à l'extrémité avant de l'appendice (1c), est rabattu vers l'intérieur par-dessus le disque (17c).

8. Dispositif suivant l'une des revendications précédentes, caractérisé en ce que derrière le bouchon d'obturation (17, 17c) est disposée une feuille (7, 24) obturant le passage.

9. Dispositif suivant la revendication 8, caractérisé en ce que la feuille (24) est réalisée en une pièce avec l'appendice (1c).

# Fig.1

# Fig. 2

# Fig.3

27 26 17a 4a 1a 22 4a 5a

IV IV

# Fig.4

4a 22 1a 4a 5a

Fig.5

Fig.6

## Fig. 7

## Fig.8